# EUROPÄISCHE PATENTSCHRIFT

(11) **EP 0 712 391 B1**
(45) Veröffentlichungstag und Bekanntmachung des Hinweises auf die Patenterteilung: **08.04.1998**
(21) Anmeldenummer: 94924285.3
(22) Anmeldetag: 25.07.1994
(51) Int. Cl.: C07C 303/20, C07C 303/32, C07C 309/12, C11D 3/37, C11D 1/12, B01F 17/00

(54) **SULFOBERNSTEINSÄUREDIESTER ENTHALTENDE FLÜSSIGE FORMULIERUNGEN**
LIQUID COMPOSITIONS CONTAINING A SULPHOSUCCINIC ACID DIESTER
FORMULATIONS LIQUIDES RENFERMANT UN DIESTER DE L'ACIDE SULFOSUCCINIQUE

(30) Priorität: 02.08.1993 DE 4325923
(43) Veröffentlichungstag der Anmeldung: 22.05.1996
(73) Patentinhaber: Henkel Kommanditgesellschaft auf Aktien, 40191 Düsseldorf (DE)
(72) Erfinder: FEUSTEL, Dieter, D-40789 Monheim (DE); HELD, Uwe, D-42553 Velbert (DE); MEYER, Joachim, D-40721 Hilden (DE)
(86) Internationale Anmeldenummer: EP9402450
(87) Internationale Veröffentlichungsnummer: WO9504035

(56) Entgegenhaltungen:
- EP-A- 0 024 711
- DE-A- 2 551 111

## Beschreibung

Die Erfindung betrifft flüssige Formulierungen, die einen Sulfobernsteinsäurediester enthalten, ein Verfahren zu deren Herstellung und die Verwendung dieser flüssigen Formulierungen als Netz- und Emulgiermittel.

Diester der Sulfobernsteinsäure sind seit vielen Jahren als wirksame Netzmittel bekannt. Im Zuge der Umstellung von organischen Lösungsmitteln enthaltenden Systeme auf wässrige Systeme hat diese Verbindungsklasse zusätzlich an Bedeutung gewonnen. Die Synthese der Diester ist auf den ersten Blick sehr einfach, was sich bereits frühzeitig in Patentliteratur niedergeschlagen hat. So beschreiben die US-PS-2 176 423, US-PS-2 416 254 und die US-PS-2 028 091, die Herstellung fester Sulfobernsteinsäurediester nach Methoden, die großtechnisch nicht realisierbar und unter wirtschaftlichen Aspekten nicht vertretbar sind.

Die Druckschriften GB-PS-1 050 578, FR-PS-1 573 080, GB-PS-760 121 und US-PS-2 879 214 beschreiben die Herstellung von flüssigen Formulierungen von Sulfobernsteinsäurediester mit großtechnisch realisierbaren Rezepturen. Hierbei wird jedoch deutlich, daß mehrere Besonderheiten die Reaktion prägen und gezielte Maßnahmen zu ihrer Beeinflussung erforderlich sind. Dabei wird weniger die Synthese der Maleinsäurediester, die als Rohstoffe in der SulfobernsteinsäurediesterSynthese eingesetzt werden, als die Sulfitierung dieses Rohstoffes zur sulfonierten Komponente als problematisch angesehen. Eine Übersicht über die Sulfitierung von Maleinsäurediestern findet sich in Anionic Surfactants, Part II, W.M. Linfield, M. Dekker, 1976, Seite 406 ff. und im Handbook of Surfactants, M.R. Porter, Blackie, 1991, Seite 107 ff.

Aus der Literatur wird deutlich, daß die Problematik der Sulfitierung in der Umsetzung eines hydrophoben Diesters mit einem wasserlöslichem HSO₃⁻ und in der Bildung von Gelphasen liegt. Es wird an verschiedenen Stellen beschrieben, daß die Reaktion nur sehr langsam anläuft, aber nach der Anlaufphase mit einer deutlichen Exothermie und schwer kontrollierbar abläuft. Um die Reaktion durchführen zu können, wird in der GB-PS-1 050 578 der Einsatz eines Initiators empfohlen. Der Initiator kann ein Tensid, insbesondere der herzustellende Sulfobernsteinsäurediester, oder ein Lösungsmittel wie Ethanol sein. Gleichzeitig wird ein organisches Lösungsmittel wie Mineralöl, Weißöl, Paraffin oder aromatische Lösungsmittel eingesetzt. In der FR-PS-1 573 080 wird die Zudosierung der Sulfitlösung beschrieben, um die Exothermie der Reaktion besser kontrollieren zu können. Gleichzeitig wird die Möglichkeit genannt, die Reaktion unter Druck durchzuführen, was Reaktionstemperaturen über dem Siedepunkt der Mischung erlaubt und den verlust an Sulfit durch Abdampfen von SO₂ verhindert. Als Vorteil wird eine kurze Reaktionszeit genannt. In der US-PS-2 879 214 wird die Bestrahlung der Reaktionsmischung mit UV-Licht empfohlen, um die Reaktion zu beschleunigen. Der Einsatz von Ethanol wird explizit oder ohne besonderen Hinweis in den Patenten FR-PS-1 573 080, GB-PS-760 121 und US-PS-2 879 214 beschrieben.

Der Einsatz von flüchtigen organischen Lösungsmitteln oder Lösungsvermittlern wie Ethanol ist erfahrungsgemäß erforderlich, um eine zügige und kontrollierbare Sulfitierungsreaktion zu gewährleisten. Außerdem werden Lösungsmittel für die Formulierung hochkonzentrierter flüssiger Sulfobernsteinsäurediester-Formulierungen benötigt. Die Diester der Sulfobernsteinsäure besitzen nur eine geringe Löslichkeit in Wasser und bilden zudem Gelphasen im mittleren Konzentrationsbereichen aus. Durch Zusatz der Colösungsmittel bleiben die flüssigen Formulierungen von Sulfobernsteinsäurediestern homogen und flüssig, was einen deutlichen Handling-Vorteil mit sich bringt.

Andererseits ist ein Gehalt an Lösungsmitteln im Hinblick auf die Emission und den Flammpunkt unerwünscht. Bei der Entwicklung lösungsmittelfreier Systeme im Lacke- und Farbenbereich wird vermehrt auf möglichst geringen Gehalt an VOC (Volatile Organic Components) geachtet. Für die Herstellung, Lagerung und den Transport ist ein möglichst hoher Flammpunkt vorteilhaft.

Dementsprechend ist es eine erste Aufgabe der vorliegenden Erfindung, flüssige, homogene, klare und einen Sulfobernsteinsäurediester enthaltende Formulierungen bereitzustellen, die Lösungsmittel enthalten, die einen hohen oder keinen Flammpunkt besitzen. Flammpunkte von wenigstens 55°C werden im Sinne der Erfindung als hoch bezeichnet. Die flüssigen Formulierungen sollen dabei ferner lagerstabil und gut handhabbar sein.

Diese Aufgabe wird erfindungsgemäß gelöst durch flüssige Formulierungen enthaltend einen Sulfobernsteinsäurediester der allgemeinen Formeln 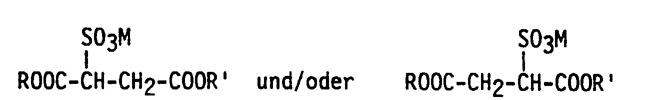 worin M ein anorganisches oder organisches Kation ist, R und R' unabhängig voneinander aliphatisches oder cycloaliphatisches C₃-C₂₂-Alkyl, C₂-C₂₂-Alkenyl oder gegebenenfalls C₁-C₈-Alkyl substituiertes C₆-C₂₂-Aryl ist, in Wasser als Lösungsmittel und einem Colösungsmittel, dadurch gekennzeichnet, daß das Colösungsmittel ein bei 10°C flüssiges, Alkylenoxideinheiten enthaltendes Polymer ist.

Gemäß der vorliegenden Erfindung können R und R' unabhängig voneinander ausgewählt sein aus (CH₃)₂CH-, C₄H₉-, C₂H₅CH(CH₃)-, C₅H₁₁-, C₃H₇CH(CH₃)-, C₆H₁₃-, C₄H₉CH(C₂H₅)CH₂-, C₈H₁₇-, C₆H₁₃CH(CH₃)-, C₇H₁₅CH(CH₃)-, C₅H₁₁CH(C₂H₅)CH₂-, CH₃C(CH₃)₂CH₂CH(CH₃)CH₂CH₂-, C₁₀H₂₁-, C₈H₁₇CH(CH₃)-, (C₄H₉)₂CHCH₂-, CH₃CH(CH₃)-(CH₂)₃-CH(CH₃)(CH₂)₂-, C₄H₉CH(C₂H₅)CH₂CH₂CH(CH₃)-, C₁₂H₂₅-, C₆H₁₃CH(C₄H₉)CH₂-, (CH₃)₂CHCH₂CH(CH₃CH₂CHCH₂CH(CH₃)₂)₂-, C₁₃H₂₇-, C₄H₉CH(C₂H₅)CH₂CH=CHCH₂CH(CH₃)CH₂-, C₁₈H₃₇-, C₈H₁₇CH=CHC₈H₁₆-, p-(CH₃)₃C-cyclo-C₆H₁₀-, p-C₂H₅C(CH₃)₂-cyclo-C₆H₁₀-, p-C₄H₉CH(CH₃)-cyclo-C₆H₁₀CH₂-, CH₃C₆H₄-.

Als Kation M eignen sich vorzugsweise solche der Alkalimetalle und insbesondere Lithium, Natrium, Kalium.

Als geeignete organische Kationen sind solche zu erwähnen, die ein quarterniertes Stickstoffatom enthalten, wie insbesondere Ammonium.

Bei den Colösungsmittel für die Sulfobernsteinsäurediester handelt es sich vorzugsweise um Blockpolymere aus Ethylenoxid (EO) und Propylenoxid (PO), die wenigstens 20 Mol-% PO enthalten. Der Anteil von PO kann 90 Mol-% betragen, liegt jedoch vorzugsweise zwischen 50 und 80 Mol-%. Das Molekulargewicht dieser EO/PO-Blockpolymere liegt zwischen 500 und 20 000, vorzugsweise zwischen 500 und 5 000.

Derartige Blockpolymere und deren Herstellung sind im Stand der Technik bekannt. Ergänzend wird jedoch auf Ullmanns Encyklopädie der technischen Chemie, 4. Auflage (1980) Band 19, Seite 31 ff. verwiesen.

Die Blockpolymere aus EO und PO können zusätzlich verestert oder verethert sein. Geeignete Ester-/Ethergruppenreste enthalten 1 bis 22 Kohlenstoffatome und können geradkettig, verzweigt oder zyklisch sein. In Frage kommen jedoch vorzugsweise die Ethan-, Propan- und Butansäureester oder die Methyl-, Ethyl-, Propyl-, Isopropyl-, Butyl-, Isobutyl- oder tert.-Butylether der Blockpolymere. Verfahren zum Endgruppenverschluß durch Veretherung oder Veresterung sind dem Fachmann aus der Literatur bekannt.

Als flüssiges, Alkylenoxideinheiten enthaltendes Polymer, das als Colösungsmittel für die Sulfobernsteinsäurediester geeignet ist, kommen Additionsprodukte von EO und/oder PO an mono- oder polyfunktionelle Alkohole in Frage. Als polyfunktionelle Alkohole sind hierbei insbesondere Ethylenglykol, Propylenglykol, Glycerin, Polyethylenglykol (PEG) und Polypropylenglykol (PPG) zu nennen.

Allgemein ist die erfindungsgemäße flüssige Formulierung dadurch gekennzeichnet, daß das flüssige Polymere in der Formulierung in einem Anteil von 1 bis 40 Gew.-%, vorzugsweise von 5 bis 20 Gew.-%, bezogen auf den Sulfobernsteinsäurediester, enthalten, ist und daß der Sulfobernsteinsäurediester in einem Anteil bis zu 70 Gew.-%, vorzugsweise zu 30 bis 60 Gew.-%, in dem Gemisch aus Wasser und dem flüssigen Polymeren vorliegt.

Besonders bevorzugte flüssige Formulierungen gemäß der Erfindung enthalten Didecylsulfosuccinat, Diisodecylsulfosuccinat, Di-2-ethylhexylsulfosuccinat oder Diisotridecylsulfosuccinat als Sulfobernsteinsäurediester. Das bevorzugte Kation ist hierbei jeweils das Natrium-Kation.

Derartige, erfindungsgemäße Formulierungen weisen einen hohen oder keinen Flammpunkt auf, sind homogen und klar und gut handhabbar. Darüber hinaus zeichnen sie sich durch eine sehr gute Lagerstabilität aus. Diese Lagerstabilität macht sich insbesondere dadurch bemerkbar, daß die Bildung einer Haut auf der Lösung vermieden wird, die durch das Verdampfen von niederen Alkoholen während der Lagerung entstehen kann.

Wie bereits erwähnt, ist die Herstellung der Diester der Sulfobernsteinsäure nicht unproblematisch und führt bei 3 bis 8 Kohlenstoffatome enthaltenden Gruppen R und R' bei der Abkühlung auf Raumtemperatur und bei 9 bis 22 Kohlenstoffatome enthaltenden Gruppen R und R' bereits während der Reaktion bei erhöhter Temperatur zur Bildung von Gelphasen, wenn ausschließlich in Wasser als Lösungsmittel gearbeitet wird. Daher wurde bisher in Gegenwart von niedrigen Alkoholen, insbesondere Ethanol oder Isopropanol als Colösungsmittel gearbeitet, wodurch die Bildung von Gelphasen weitestgehend vermieden werden kann.

Insbesondere im Hinblick auf die Anforderung auch in Formulierungen flüchtige organische Lösungsmittel zu vermeiden und auch bereits während der Herstellung Lösungsmittel einzusetzen, die einen hohen oder keinen Flammpunkt aufweisen, war es eine weitere Aufgabe der vorliegenden Erfindung ein praktikables Verfahren zur Herstellung der erfindungsgemäßen Formulierungen zur Verfügung zu stellen, welches es ermöglicht, direkt die erfindungsgemäßen flüssige Formulierungen von Sulfobernsteinsäurediestern herzustellen, die weder während der Reaktion der Ausgangsverbindungen zu den Sulfosuccinaten noch beim Abkühlen des Reaktionsgemisches auf Raumtemperatur zu Gelphasenbildung neigen.

Diese Aufgabe wurde erfindungsgemäß gelöst durch ein Verfahren zur Herstellung der oben beschriebenen Sulfobernsteinsäurediester enthaltenden flüssigen Formulierungen aus einem Maleinsäurediester der allgemeinen Formel ROOC-CH=CH-COOR', worin R, R' unabhängig voneinander aliphatisches oder cycloaliphatisches C₃-C₂₂-Alkyl, C₂-C₂₂-Alkenyl oder gegebenenfalls C₁-C₈-Alkyl substituiertes C₆-C₂₂-Aryl ist und einem geeigneten Sulfitierungsagens in Wasser als Lösungsmittel in Gegenwart eines flüssigen, wasserlöslichen Colösungsmittels in einem Temperaturbereich von 60 bis 150°C gegebenenfalls unter Überdruck, wobei, wenn R und R' C₃-C₈-Alkyl ist, das Colösungsmittel nach der Sulfitierungsreaktion und vor der Abkühlung dem Reaktionsgemisch zugegeben werden kann.

Die Diester mit kurzkettigen bis 8 Kohlenstoffatome enthaltenden Alkoholen wie Isobutanol, Isoamylalkohol oder 2 Ethylhexanol lassen sich unter geeigneten Reaktionsbedingungen ohne den Einsatz eines Colösungsmittels synthetisieren. Um jedoch auch bei Abkühlung des Reaktionsgemisches auf Raumtemperatur homogene und flüssige Produkte zu erhalten und um die Bildung von Gelphasen zu vermeiden, ist jedoch der Einsatz der erfindungsgemäßen Colösungsmittel erforderlich. Die Sulfobernsteinsäurediester der längerkettigen 9 bis 22 Kohlenstoffatome enthaltenden Alkohole werden jedoch bereits in Gegenwart der flüssigen Colösungsmittel hergestellt, da sich auch bei erhöhter Reaktionstemperatur die Bildung von Gelphasen in Wasser als alleinigem Lösungsmittel nicht vermeiden läßt.

Hinsichtlich der erfindungsgemäß einzusetzenden flüssigen Polymere als Colösungsmittel sei auf das oben Ausgeführte verwiesen.

In den erfindungsgemäß eingesetzten Maleinsäurediestern der Formel ROOC-CH=CH-COOR' haben die Gruppen R und R' die oben angeführte Bedeutung. Die Herstellung dieser Maleinsäurediester erfolgt nach im Stand der Technik bekannten Verfahrensweisen. Ergänzend hierzu sei auf Römpp Chemie Lexikon, 9. Auflage (1991), Band 4, Seite 2617 und die dort referierte Literatur verwiesen.

Bei den Sulfitierungsagenzien handelt es sich um bei Sulfitierungsreaktionen üblicherweise eingesetzte Agenzien, insbesondere MHSO₃, M₂SO₃ oder M₂S₂O₅, wobei M für ein wie oben beschriebenes anorganisches oder organisches Kation steht.

Das molare Verhältnis von Maleinsäurediester zu dem Sulfitierungsagens (bezogen auf HSO₃⁻) beträgt zwischen 1:0,9 bis 1:2, vorzugsweise 1:1 bis 1:1,5 und besonders bevorzugt 1:1 bis 1:1,2.

Die Reaktionstemperatur beträgt vorzugsweise zwischen 60 und 150°C wobei, wenn höhere Reaktionstemperaturen als 100°C angewandt werden sollen, in geeigneten Apparaturen unter Überdruck gearbeitet werden muß. Die Obergrenze der Reaktionstemperatur ist hierbei durch die Isomerisierungstemperatur von Maleinsäure zu Fumarsäure gegeben, die bei etwa 150°C liegt.

Es hat sich ferner gezeigt, daß es vorteilhaft sein kann, die Sulfitierungsreaktion des Maleinsäurediesters in Gegenwart eines Tensides, vorzugsweise des herzustellenden Sulfobernsteinsäurediestersalzes als Initiator durchzuführen. Dies bewirkt, daß die Reaktion von Anfang an besser kontrollierbar und steuerbar wird, da die Reaktion ansonsten erst nach einiger Zeit bedingt durch Autokatalyse in Gang kommt. Der Initiator kann dem Reaktionsgemisch in einer Menge von 0,1 bis 20 Gew.-%, vorzugsweise 0,1 bis 15 Gew.-% bezogen auf den Maleinsäurediester zugesetzt werden.

Zur Durchführung der Reaktion wird der Anteil des Wassers und des Colösungsmittels im Reaktionsgemisch so berechnet, daß das flüssige Polymere in einem Anteil von 1 bis 40 Gew.-%, vorzugsweise 5 bis 20 Gew.-%, bezogen auf das gewünschte Sulfobernsteinsäurediester Reaktionsprodukt eingesetzt wird. Für praktische Anwendungen ist es zweckmäßig, daß die Anteile von Wasser und Colösungsmittel so aufeinander abgestimmt werden, daß der gewünschte Sulfobernsteinsäurediester als bis 70 gew-.%ige, vorzugsweise 30 bis 60 gew.-%ige Lösung in dem Lösungsmittel/Colösungsmittelgemisch vorliegt.

Das oben genannte Verfahren hat insgesamt den Vorteil, daß stets in einer klaren und homogenen Lösung gearbeitet werden kann, ohne daß mit flüchtigen organischen Lösungsmitteln, die einen niedrigen Flammpunkt besitzen, gearbeitet werden muß. Die Bildung von Gelphasen kann durch diese Verfahrensweise vollständig unterbunden werden.

Die erfindungsgemäßen flüssigen Formulierungen finden insbesondere als Netz- und Emulgiermittel Anwendung. Die erfindungsgemäßen flüssigen Formulierungen zeichnen sich insbesondere durch ihre Verträglichkeit bei der Anwendung als Netzmittel in Polymerdispersionen aus. Dies macht sich vor allem dadurch bemerkbar, daß insbesondere die Sulfobernsteinsäurediester von längerkettigen, mehr als jeweils 8 Kohlenstoffe enthaltende Estergruppen, bei der Anwendung nicht zur Trübung durch Ausfällungen führen.

Grundsätzlich eignen sich jedoch die erfindungsgemäßen flüssigen Formulierungen für eine Vielzahl von typischen Anwendungen, wie sie insbesondere in der US-PS-2 176 423 aufgezählt sind. Bevorzugt sind jedoch Anwendungen der erfindungsgemäßen Formulierungen in wässrigen Farben, Druckfarben und Überdrucklacken.

Die nachfolgenden Beispiele dienen der Erläuterung der vorliegenden Erfindung, sind jedoch nicht einschränkend zu verstehen.

### Beispiele

### Beispiel 1:

### Synthese von Di-2-ethylhexylsulfosuccinat

295,5 g Di-2-ethylhexylmaleinat, 25 g Di-2-ethylhexylsulfosuccinat, Na-Salz (75 %ig in Wasser), 85,5 g Natriumdisulfit Pulver und 190,1 g Wasser wurden in einem Dreihalskolben mit Rückflußkühler, Rührer, Thermometer und Stickstoffeinleitung vorgelegt auf 102°C erhitzt. Nach ca. 15 Minuten trat ein leichter Rückfluß auf, der im Laufe der Reaktion stärker wurde. Die Temperatur wurde bei 102°C gehalten, bis der Rückfluß vollkommen abklang. Anschließend wurde bis zur Sulfitfreiheit nachgerührt.

### Beispiel 1 a) (Vergleichsbeispiel):

Die Reaktionsmischung wurde auf 80°C abgekühlt und 27 g Ethanol zugegeben. Anschließend wurde auf Raumtemperatur abgekühlt. Man erhielt ein klares, homogenes, flüssiges Produkt mit ca. 70 % Aktivsubstanz. Der Flammpunkt des Produkts war < 55°C.

### Beispiel 1 b) (Vergleichsbeispiel):

35 g 1,2-Propylenglykol wurden zu der Reaktionsmischung gegeben. Anschließend wurde der Ansatz auf Raumtemperatur abgekühlt. Man erhielt ein klares, homogenes, flüssiges Produkt mit ca. 70 % Aktivsubstanz. Der Flammpunkt des Produkts war > 55° C.

### Beispiel 1 c):

360 g Wasser und 100 g eines Blockpolymeren aus Ethylenoxid und Propylenoxid mit einer Molmasse von ca. 2 000 und einem PO-Anteil von 75 Mol-% (EO/PO-Blockpolymer aus 25 Mol PO und 11 Mol EO auf Propylenglykol) wurden zu der Reaktionsmischung gegeben. Anschließend wurde auf Raumtemperatur abgekühlt. Man erhielt ein klares, mittelviskoses, flüssiges Produkt mit ca. 50 % Aktivsubstanz, das einen Flammpunkt von > 100°C aufweist. Ein gleiches Ergebnis erhielt man, wenn man anstelle des oben genannten Blockpolymeren ein EO/PO Blockpolymeres aus 30 Mol PO und 5 Mol EO auf Propylenglykol einsetzte.

### Beispiel 2 (Vergleichsbeispiel):

### Synthese von Diisodecylsulfosuccinat mit Ethanol als Colösungsmittel

533 g Diisodecylmaleinat, 50 g Diisodecylsulfosuccinat Na-Salz, 132 g Natriumdisulfit Pulver, 190 g Wasser und 95 g Ethanol wurden in einem Dreihalskolben mit Rückflußkühler, Rührer, Thermometer und Stickstoffeinleitung vorgelegt. Die Mischung wurde auf 85°C erhitzt worauf ein Rückfluß eintrat. Sobald der Rückfluß schwächer wurde, wurde in mehreren Schritten die Temperatur auf 90°C erhöht. Bei dieser Temperatur wurde gerührt, bis der Rückfluß zum Erliegen kam. Anschließend wurde bis zur Sulfitfreiheit nachgerührt und auf Raumtemperatur abgekühlt.

Man erhielt ein klares, homogenes, flüssiges Produkt mit ca. 70 % Aktivsubstanz. Das Produkt wies einen Flammpunkt < 55°C auf.

### BeisDiel 3 (Vergleichsbeispiel):

### Synthese von Diisodecylsulfosuccinat ohne Ethanol als Colösungsmittel

533 g Diisodecylmaleinat, 50 g Diisodecylsulfosuccinat, Na-Salz, 132 g Natriumdisulfit Pulver und 185 g Wasser wurden in einer Apparatur analog Beispiel (2) vorgelegt. Die Mischung wurde auf 102°C erwärmt, wobei ein Rückfluß und starke Schaumbildung auftrat. Die Reaktion wurde bei 98°C fortgesetzt. Nach ca. 3 Stunden Reaktionszeit war die Viskosität so stark angestiegen, daß die Mischung nicht mehr gerührt werden konnte. Nach Abkühlung lag ein unvollständig umgesetztes, festes Produkt vor, das nur durch Zugabe von 500 ml Ethanol langsam aufgelöst werden konnte.

### Beispiel 4:

### Synthese von Diisodecylsulfosuccinat in Gegenwart eines wasserlöslichen Polymeren des EO/PO-Blockpolymeren-Typs als Colösungsmittel

87,8 g Diisodecylmaleinat, 15 g Diisodecylsulfosuccinat Na-Salz, 21,7 g Natriumdisulfit Pulver, 52 g Wasser und 30 g eines EO/PO-Blockpolymeren (MG ca. 2 000, PO-Anteil 75 Mol-% (EO/PO-Blockpolymer aus 25 Mol PO und 11 Mol EO auf Propylenglykol) wurden in einer Apparatur analog Beispiel (1) vorgelegt. Die Mischung wurde auf 102°C erwärmt. Nach ca. 30 Minuten setzte ein schwacher Rückfluß ein. Die Reaktion wurde bei 103°C fortgesetzt. Nach ca. 5 Stunden Reaktionszeit klang der Rückfluß ab. Es wurde bei konstanter Temperatur bis zur Sulfitfreiheit weitergerührt. Anschließend wurde auf Raumtemperatur abgekühlt.

Man erhielt ein klares, homogenes, flüssiges Produkt mit ca. 75 % Aktivsubstanz. Das Produkt wies einen Flammpunkt von > 100°C auf. Ein gleiches Ergebnis erhielt man, wenn man anstelle des oben genannten Blockpolymeren ein EO/PO Blockpolymeres aus 30 Mol PO und 5 Mol EO auf Propylenglykol einsetzte.

## Patentansprüche

1. Flüssige Formulierung enthaltend einen Sulfobernsteinsäurediester der allgemeinen Formeln 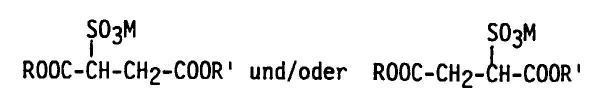 worin M ein anorganisches oder organisches Kation ist, R und R' unabhängig voneinander aliphatisches oder cycloaliphatisches C₃-C₂₂-Alkyl, C₂-C₂₂-Alkenyl oder gegebenenfalls C₁-C₈-Alkyl substituiertes C₆-C₂₂-Aryl ist, in Wasser als Lösungsmittel und einem Colösungsmittel, dadurch gekennzeichnet, daß das Colösungsmittel ein bei 10°C flüssiges Alkylenoxideinheiten enthaltendes Polymer ist.

2. Flüssige Formulierung nach Anspruch 1, dadurch gekennzeichnet, daß das Colösungsmittel ausgewählt ist aus einem Blockpolymeren aus Ethylenoxid (EO) und Propylenoxid (PO) oder Additionsprodukten von EO und/oder PO an mono- oder polyfunktionelle Alkohole.

3. Flüssige Formulierung nach Anspruch 2, dadurch gekennzeichnet, daß der polyfunktionelle Alkohol Ethylenglykol oder Propylenglykol ist.

4. Flüssige Formulierung nach Anspruch 2 oder 3, dadurch gekennzeichnet, daß das Blockpolymere aus EO und PO einen PO-Anteil von wenigstens 20 Mol-% enthält.

5. Flüssige Formulierung nach Anspruch 2 oder 4, dadurch gekennzeichnet, daß das Blockpolymere aus EO und PO ein Molekulargewicht von 500 bis 20 000 besitzt.

6. Flüssige Formulierung nach einem oder mehreren der Ansprüche 1 bis 5, dadurch gekennzeichnet, daß das flüssige Alkylenoxideinheiten enthaltende Polymere in der Formulierung in einem Anteil von 1-40 Gew.-%, bezogen auf den Sulfobernsteinsäurediester, enthalten ist.

7. Flüssige Formulierung nach einem oder mehreren der Ansprüche 1 bis 5, dadurch gekennzeichnet, daß der Sulfobernsteinsäurediester in einem Anteil von bis zu 70 Gew.-% in dem Gemisch aus Wasser und dem flüssigen Polymeren vorliegt.

8. Flüssige Formulierung nach einem oder mehreren der Ansprüche 1 bis 6, dadurch gekennzeichnet, daß der Sulfobernsteinsäurediester Didecylsulfosuccinat, Diisodecylsulfosuccinat, Di-2-ethylhexylsulfosuccinat oder Diisotridecylsulfosuccinat ist.

9. Verfahren zur Herstellung der flüssigen Formulierungen, wie in einem oder mehreren der Ansprüche 1 bis 8 definiert, aus einem Maleinsäurediester der allgemeinen Formel ROOC-CH=CH-COOR', worin R, R' unabhängig voneinander aliphatisches oder cycloaliphatisches C₃-C₂₂-Alkyl, C₂₋C₂₂-Alkenyl oder gegebenenfalls C₁-C₈-Alkyl substituiertes C₆-C₂₂-Aryl ist, und einem geeigneten Sulfitierungsagens in Wasser als Lösungsmittel in Gegenwart eines flüssigen Colösungsmittels in einem Temperaturbereich von 60 bis 150°C gegebenenfalls unter Überdruck, wobei, wenn R und R' C₃-C₈-Alkyl ist, das Colösungsmittel nach der Reaktion und vor der Abkühlung dem Reaktionsgemisch zugegeben werden kann.

10. Verfahren nach Anspruch 9, dadurch gekennzeichnet, daß das molare Verhältnis von Maleinsäurediester zu Sulfitierungsagens (bezogen auf HSO₃⁻) 1:0,9 bis 1:2 beträgt.

11. Verfahren nach Anspruch 9 oder 10, dadurch gekennzeichnet, daß das Sulfitierungsagens MHSO₃, M₂SO₃ oder M₂S₂O₅ ist, wobei M für ein anorganisches oder organisches Kation steht.

12. Verfahren nach einem oder mehreren der Ansprüche 9 bis 11, dadurch gekennzeichnet, daß man das herzustellende Produktes als Initiator einsetzt.

13. Verfahren nach einem oder mehreren der Ansprüche 9 bis 12, dadurch gekennzeichnet, daß das flüssige Polymere ein Blockpolymeres aus Ethylenoxid (EO) und Propylenoxid (PO) oder ein Additionsprodukt von EO und/oder PO an mono- oder polyfunktionelle Alkohole ist.

14. Verfahren nach Anspruch 13, dadurch gekennzeichnet, daß der polyfunktionelle Alkohol Ethylenglykol oder Propylenglykol ist.

15. Verwendung der flüssigen Formulierung nach einem der Ansprüche 1 bis 8 als Netz- bzw. Emulgiermittel.

## Claims

1. A liquid formulation containing a sulfosuccinic acid diester corresponding to the following general formulae: 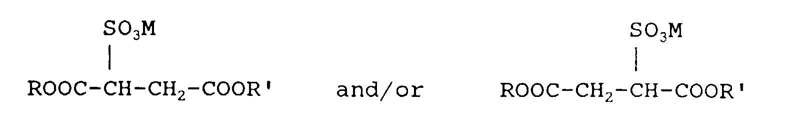 in which M is an inorganic or organic cation, R and R' independently of one another represent aliphatic or cycloaliphatic C₃₋₂₂ alkyl, C₂₋₂₂ alkenyl or optionally C₁₋₈ alkyl-substituted C₆₋₂₂ aryl, in water as solvent and a co-solvent, characterized in that the co-solvent is a polymer containing alkylene oxide units which is liquid at 10°C.

2. A liquid formulation as claimed in claim 1, characterized in that the co-solvent is selected from a block polymer of ethylene oxide (EO) and propylene oxide (PO) or adducts of EO and/or PO with monofunctional or polyfunctional alcohols.

3. A liquid formulation as claimed in claim 2, characterized in that the polyfunctional alcohol is ethylene glycol or propylene glycol.

4. A liquid formulation as claimed in claim 2 or 3, characterized in that the block polymer of EO and PO has a percentage PO content of at least 20 mole-%.

5. A liquid formulation as claimed in claim 2 or 4, characterized in that the block polymer of EO and PO has a molecular weight of 500 to 20,000.

6. A liquid formulation as claimed in one or more of claims 1 to 5, characterized in that the liquid polymer containing alkylene oxide units is present in the formulation in a quantity of 1 to 40% by weight, based on the sulfosuccinic acid diester.

7. A liquid formulation as claimed in one or more of claims 1 to 5, characterized in that the sulfosuccinic acid diester is present in the mixture of water and the liquid polymer in a quantity of up to 70% by weight.

8. A liquid formulation as claimed in one or more of claims 1 to 6, characterized in that the sulfosuccinic acid diester is didecyl sulfosuccinate, diisodecyl sulfosuccinate, di-2-ethylhexyl sulfosuccinate or diisotridecyl sulfosuccinate.

9. A process for the production of the liquid formulations claimed in one or more of claims 1 to 8 from a maleic acid diester corresponding to the general formula ROOC-CH=CH-COOR', where R and R' independently of one another represent aliphatic or cycloaliphatic C₃₋₂₂ alkyl, C₂₋₂₂ alkenyl or optionally C₁₋₈-alkyl-substituted C₆₋₂₂ aryl, and a suitable sulfiting agent in water as solvent in the presence of a liquid co-solvent at temperatures of 60 to 150°C and optionally under excess pressure; where R and R' represent C₃₋₈ alkyl, the co-solvent may be added to the reaction mixture after the sulfitation reaction and before cooling.

10. A process as claimed in claim 9, characterized in that the molar ratio of maleic acid diester to sulfiting agent (based on HSO₃₋) is 1:0.9 to 1:2.

11. A process as claimed in claim 9 or 10, characterized in that the sulfiting agent is MHSO₃, M₂SO₃ or M₂S₂O₅, where M is an inorganic or organic cation.

12. A process as claimed in one or more of claims 9 to 11, characterized in that the product to be produced is used as an initiator.

13. A process as claimed in one or more of claims 9 to 12, characterized in that the liquid polymer is a block polymer of ethylene oxide (EO) and propylene oxide (PO) or an addition product of EO and/or PO with monofunctional or polyfunctional alcohols.

14. A process as claimed in claim 13, characterized in that the polyfunctional alcohol is ethylene glycol or propylene glycol.

15. The use of the liquid formulation claimed in any of claims 1 to 8 as a wetting agent or emulsifier.

## Revendications

1. Formulation liquide contenant un diester de l'acide sulfosuccinique de formules générales 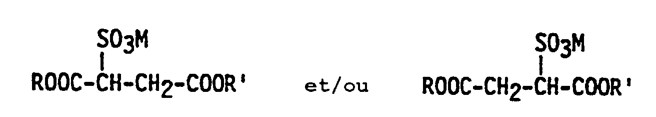 dans lesquelles M est un cation minéral ou organique, R et R' représentent indépendamment l'un de l'autre un alkyle en C₃ à C₂₂ aliphatique ou cyclo-aliphatique, un alcényle en C₂ à C₂₂ ou un aryle en C₃ à C₂₂ éventuellement substitué par un alkyle en C₁ à C₈, dans l'eau comme solvant et un cosolvant,
caractérisée en ce que
le cosolvant est un polymère contenant des unités oxyde d'alkylène liquide à 10°C.

2. Formulation liquide selon la revendication 1,
caractérisée en ce que
le cosolvant est choisi à partir d'un polymère séquencé d'oxyde d'éthylène (OE) et d'oxyde de propylène (OP) ou de produits d'addition d'OE et/ou d'OP sur des alcools mono- ou polyfonctionnels.

3. Formulation liquide selon la revendication 2,
caractérisée en ce que
l'alcool polyfonctionnel est l'éthylène glycol ou le propylène glycol.

4. Formulation liquide selon les revendications 2 ou 3,
caractérisée en ce que
le polymère séquencé d'OE et d'OP contient une proportion d'OP d'au moins 20% molaire.

5. Formulation liquide selon les revendications 2 ou 4,
caractérisée en ce que
le polymère séquencé d'OE et d'OP possède un poids moléculaire de 500 à 20 000.

6. Formulation liquide selon une ou plusieurs des revendications 1 à 5,
caractérisée en ce que
le polymère contenant des unités oxyde d'alkylène liquide est contenu dans la formulation en une proportion de 1-40% en poids, par rapport au diester de l'acide sulfosuccinique.

7. Formulation liquide selon une ou plusieurs des revendications 1 à 5,
caractérisée en ce que
le diester de l'acide sulfosuccinique est présent en une proportion allant jusqu'à 70% en poids dans le mélange d'eau et du polymère liquide.

8. Formulation liquide selon une ou plusieurs des revendications 1 à 6,
caractérisée en ce que
le diester de l'acide sulfosuccinique est le sulfosuccinate de didécyle, le sulfosuccinate de diisodécyle, le sulfosuccinate de di-2-éthylhexyle ou le sulfosuccinate de diisotridécyle.

9. Procédé de fabrication des formulations liquides, tel que défini dans une ou plusieurs des revendications 1 à 8, à partir d'un diester de l'acide maléique de formule générale ROOC-CH=CH-COOR', dans laquelle R et R' représentent indépendamment l'un de l'autre un alkyle en C₃ à C₂₂ aliphatique ou cycloaliphatique, un alcényle en C₂ à C₂₂ ou un aryle en C₃ à C₂₂ éventuellement substitué par un alkyle en C₁ à C₈, et d'un agent de sulfitation approprié dans l'eau comme solvant en présence d'un cosolvant liquide dans un intervalle de températures allant de 60 à 150°C, le cas échéant sous une surpression, où lorsque R et R' représentent un alkyle en C₃ à C₈, le cosolvant peut être ajouté au mélange réactionnel après la réaction et avant le refroidissement.

10. Procédé selon la revendication 9,
caractérisé en ce que
le rapport molaire du diester de l'acide maléique à l'agent de sulfitation (par rapport à HSO₃⁻ ) s'élève à 1:0,9 à 1:2.

11. Procédé selon la revendication 9 ou 10, caractérisé en ce que l'agent de sulfitation est MHSO₃, M₂SO₃ ou M₂S₂O₅, où M représente un cation minéral ou organique.

12. Procédé selon une ou plusieurs des revendications 9 à 11,
caractérisé en ce qu'
on utilise comme initiateur le produit à fabriquer.

13. Procédé selon une ou plusieurs des revendications 9 à 12, caractérisé en ce que le polymère liquide est un polymère séquencé constitué d'oxyde d'éthylène (OE) et d'oxyde de propylène (OP) ou d'un produit d'addition d'OE et/ou d'OP sur des alcools mono- ou polyfonctionnels.

14. Procédé selon la revendication 13,
caractérisé en ce que
l'alcool polyfonctionnel est l'éthylène glycol ou le propylène glycol.

15. Utilisation de la formulation liquide selon l'une des revendications 1 à 8 comme agent mouillant ou selon les cas comme agent émulsifiant.
